# EUROPÄISCHE PATENTSCHRIFT

(11) **EP 0 646 374 B1**
(45) Veröffentlichungstag und Bekanntmachung des Hinweises auf die Patenterteilung: **08.04.1998**
(21) Anmeldenummer: 94810494.8
(22) Anmeldetag: 30.08.1994
(51) Int. Cl.: A61K 31/55, A61K 47/02, A61K 9/28

(54) **Doppelt beschichtete Oxcarbazepin-Tabletten**
Double-coated oxcarbazepine containing tablets
Comprimé à double revêtement contenant l'oxcarbazépine

(30) Priorität: 08.09.1993 CH 2679/93
(43) Veröffentlichungstag der Anmeldung: 05.04.1995
(73) Patentinhaber: Novartis AG, 4058 Basel (CH)
(72) Erfinder: Bourquin, Jacques, CH-4125 Riehen (CH)

(56) Entgegenhaltungen:
- BE-A- 892 882
- DE-A- 2 011 087

## Beschreibung

Die vorliegende Erfindung betrifft doppelt beschichtete Tabletten für den Wirkstoff Oxcarbazepin und Verfahren zur Herstellung dieser Tabletten.

Oxcarbazepin, 10,11-Dihydro-10-oxo-5H-dibenz[b,f]azepin-5-carboxamid, gilt wie ®Tegretol ((Ciba-Geigy) Carbamazepin: 5H-Dibenz[b,f]azepin-5-carboxamid) als Mittel der ersten Wahl bei der Behandlung von Konvulsionen und starken Schmerzzuständen. Die kommerziell erhältlichen Darreichungsformen, wie Tabletten und Sirupe, eignen sich insbesondere für regelmassig wiederkehrende Verabreichungen in einem längeren Behandlungszeitraum, um eine gleichmässige Wirkstoffkonzentration im Blut sicherzustellen. In der Belgischen Patentanmeldung 892882 sind Dragées mit dem Pigment Titandioxid in der Zuckerschicht beschrieben. Dort erfolgt kein Hinweis auf die Beschichtung von Tabletten.

Bei Tabletten mit dem Wirkstoff Oxcarbazepin lässt sich ein Stabititätsproblem feststellen: Während der Lagerung bei Raumtemperatur tritt eine uneinheitliche orangefarbene Veränderung von geringer Intensität des ursprünglich weissen Komprimats auf. Untersuchungen haben ergeben, dass diese unerwünschte Farbabweichung durch ein in geringer Menge (≤ 0,05 %) entstandenes Oxydationsprodukt des deklarierten Wirkstoffs verursacht wird. Man nimmt an, dass sich dieses Oxydationsprodukt als Diketoiminodibenzyl: 10,11-Dihydro-5H-dibenz[b,f]azepin-10,11-dion, bezeichnen lässt. Das Oxydationsprodukt sollte an sich pharmakologisch unbedenklich sein. Um aber eine möglichst lange äusserliche Homogenität des Handelsprodukts zu erhalten, mischt man beim Komprimieren das Pigment Eisen-II-oxid (FeO) hinzu, welches die uneinheitliche Orangefärbung des Oxydationsprodukts abdecken soll. Die durch diesen Zusatz von Eisen-II-oxid verursachte einheitliche gelblich-orange Einfärbung der Tablette kann hingenommen werden, da während ihrer Lagerung keine weiteren farblichen Änderungen erkennbar sind. Überhaupt sind nur sehr wenige Farbstoffe in enteralen Darreichungsformen pharmakologisch unbedenklich und daher für Formulierungszwecke zugelassen.

Allerdings ist auch die Verwendung von Eisen-II-oxid als Hilfsstoff bei der Herstellung von Tabletten problematisch. So hat in den USA die Zulassungsbehörde FDA (Food and Drug Administration) die Forderung nach einer Begrenzung der Dosierung des Hilfsstoffs erhoben, indem die Einnahme nur noch einer tägliche Höchstmenge von 5 mg Eisen zulässig ist. Es wird erwartet, dass sich die zuständigen Zulassungsbehörden in anderen Ländern dieser Forderung anschliessen werden. Ihr Hintergrund ist die physiologische Wirksamkeit von Eisen selbst, die bei einem Hilfsstoff unerwünscht ist.

Der vorliegenden Erfindung liegt die Aufgabe zugrunde, farbstabile Tabletten für den bewährten Wirkstoff Oxcarbazepin herzustellen, welche die Einhaltung einer Zufuhr von höchstens 5 mg Eisen Tagesdosis erlauben.

Diese Aufgabenstellung wird durch die vorliegende Erfindung gelöst, welche eine doppelt beschichtete Tablette für den Wirkstoff 10,11-Dihydro-10-oxo-5H-dibenz[b,f]azepin-5-carboxamid betrifft und durch folgende Bestandteile gekennzeichnet ist:
a) einen Tablettenkern enthaltend eine therapeutisch wirksame Dosis von 10,11-Dihydro-10-oxo-5H-dibenz[b,f]azepin-5-carboxamid;
b) eine hydrophile, permeable innere Beschichtung enthaltend weisse Farbpigmente und gegebenenfalls weitere Hilfsstoffe und
c) eine hydrophile, permeable äussere Beschichtung enthaltend weisse Farbpigmente kombiniert mit Eisen-II-oxid-Farbpigmenten und gegebenenfalls weitere Hilfsstoffe.

Die Tabletten gemäss vorliegender Erfindung erfüllen die Kriterien der Lagerstabilität und zeichnen sich insbesondere durch bis zu mehreren Monaten andauernde Farbstabilität aus. Die Tabletten gemäss vorliegender Erfindung bleiben sehr lange einheitlich gelblich gefärbt.

Ebenfalls Gegenstand der vorliegenden Erfindung ist das Verfahren zur Herstellung der doppelt beschichteten Tablette für den Wirkstoff 10,11-Dihydro-10-oxo-5H-dibenz[b,f]azepin-5-carboxamid. Dieses Herstellungsverfahren ist dadurch gekennzeichnet, dass man
a) den Wirkstoff unter Zumischung von üblichen Hilfsstoffen und unter Anwendung üblicher Tablettierungsverfahren zu einem Tablettenkern verpresst und diesen nacheinander mit
b) einer hydrophilen, permeablen inneren Beschichtung mit weissen Farbpigmenten und
c) einer hydrophilen, permeablen äusseren Beschichtung mit weissen Farbpigmenten kombiniert mit Eisen-II-oxid-Farbpigmenten versieht.

Die weiter vorn und im folgenden verwendeten Begriffe sind im Rahmen der Beschreibung der vorliegenden Erfindung wie folgt definiert:

Der Begriff doppelt beschichtete Tablette bezeichnet eine peroral applizierbare einzeldosierte feste Darreichungsform, welche sich durch Komprimierung des Wirkstoffs mit üblichen Tablettierungshilfsstoffen zu einem Tablettenkern unter Anwendung üblicher Tablettierungsverfahren und anschliessende Beschichtung herstellen lässt. Gemäss einer bevorzugten Verfahrensvariante werden Tablettenkerne unter Anwendung des sogenannten Kompaktierungsverfahrens (syn. Trockengranulierung) hergestellt, indem man den Wirkstoff mit den Hilfsstoffen zu grösseren Formkörpern, z.B. Schülpen, verpresst, diese durch Mahlen zerkleinert und das Mahlgut zu Tablettenkernen verpresst. Das Kompaktierungsverfahren ist z.B. in R.Voigt, Lehrbuch der Pharmazeutischen Technologie, 6.Auflage, VCH 1987, ISBN 3-527-26595-3, Seite 162, beschrieben.

Für das Kompaktierungsverfahren geeignete Hilfsstoffe sind vorzugsweise solche, welche sich für die üblichen Verfahren zur Direktverpressung eignen, z.B. Trockenbindemittel, wie Stärke, z.B. Kartoffel-, Weizen- und Maisstärke, mikrokristalline Cellulose, z.B. Handelsware, welche unter den Warenzeichen Avicel®, Filtrak®, Heweten® oder Pharmacel® kommerziell erhältlich ist, hochdisperses Siliciumdioxid, z.B. Aerosil®, Mannit, Lactose ferner Polyethylenglycol, insbesondere mit einer Molmasse von 4 000 bis 6 000, vernetztes Polyvinylpyrrolidon (Polyplasdone® XL oder Kollidon®CL), quervernetzte Carboxymethylcellulose (Acdisol® CMC-XL), Carboxymethylcellulose [Nymcel®, z.B. ZSB-10, (Nyma)], Hydroxypropylmethylcellulose, z.B. die Qualität HPMC 603, Carboxymethylstärke [Explotab® (Mendell) oder Primojel® (Scholtens)], mikrokristalline Cellulose, z.B. Avicel® PH 102, Dicalciumphosphat, z.B. Emcompress® oder Talk. Ferner sind Zusätze von geringen Mengen Schmiermittel, z.B. Magnesiumstearat, vorteilhaft.

Als Hilfsstoffe sind ferner Ultraamylopektin, silikonisierter Talk, Aluminiumstearat, Stearinsäure, Palmitinsäure, entfettetes Milchpulver, Stearyl-, Cetyl- und Myristylalkohol, Lanette®O, Paraffin oder hydrierte Fette geeignet. Auf die umfangreiche Fachliteratur zum Thema Tablettierung wird verwiesen.

So ist beispielsweise das übliche Verfahren der Nassgranulierung ebenfalls geeignet, indem man Füllstoffe, wie Stärke, mikrokristalline Cellulose, Lactose, Glucose, Mannit, Talk, Dextrin oder Alginat mit Bindemitteln wie Polyvinylpyrrolidon, Gelatine, Gummi Arabicum, Tragant oder Akaziengummi vermischt und das Gemisch mit einer wässrig äthanolischen Lösung versetzt. Man knetet die feuchte Masse, entfernt das Lösungsmittel durch Trocknung und presst die gebildeten Granulate, gegebenenfalls unter Zusatz von Fliessregulierungsmitteln, Schmiermitteln, oder Formtrennmitteln in einer Tablettiermaschine zu Tablettenkernen.

Der Wirkstoff Oxcarbazepin: 10,11-Dihydro-10-oxo-5H-dibenz[b,f]azepin-5-carboxamid, ist bekannt. Seine Herstellung und therapeutische Verwendbarkeit als Antikonvulsivum ist in der Deutschen Auslegeschrift 20 11 087 beschrieben. Ein technisch vorteilhaftes Herstellungsverfahren für diesen Wirkstoff ist in der Europäischen Patentanmeldung Nr. 0 028 028 beschrieben. Kommerziell erhältliche Darreichungsformen sind für die perorale Verabreichung vorgesehen, z.B. Tabletten mit 300 und 600 mg Wirkstoff. Diese Darreichungsformen sind unter dem Warenzeichen ®Trileptal (Ciba-Geigy) bekannt und in zahlreichen Ländern wie Dänemark, Finnland, Österreich, Belgien eingeführt worden. Die Einführung u.a. auch eines Sirups in weiteren Ländern steht unmittelbar bevor.

Tablettenkerne entstehen durch Verpressen der weiter vorn gemahlenen Formkörper, z.B. Schülpen, oder von Granulaten, z.B. Nassgranulaten in üblichen Tablettiermaschinen, z.B. EK-0 Korsch-Exzenter-Tablettiermaschinen, vorzugsweise bei einem Pressdruck grösser als 10 kN. Die Tablettenkerne können unterschiedlich geformt und z.B. rund, oval, oblong, zylindrisch u.a. sein und verschiedene Grössen in Abhängigkeit von der Wirkstoffmenge haben.

Die hydrophile, permeable innere Beschichtung b) besteht aus einem filmbildenden Material, das für Wasser bzw. Darmsaft durchlässig und in diesen Flüssigkeiten quellbar und zumindest teilweise löslich ist.

Filmbildende Materialien mit Wasserdurchlässigkeit sind z.B. hydrophile Gemische von Polyvinylpyrrolidon oder eines Copolymerisates von Polyvinylpyrrolidon und Polyvinylacetat mit Hydroxypropylmethylcellulose, Gemische von Schellack mit Hydroxypropylmethylcellulose, Polyvinylacetat oder dessen Copolymeren mit Polyvinylpyrrolidon, oder Gemische von wasserlöslichen Cellulosederivaten, wie Hydroxypropylmethylcellulose, und wasserunlöslicher Äthylcellulose.

Diese eigentlichen Beschichtungsmittel können, wenn erwünscht, im Gemisch mit anderen zusätzlichen Hilfsstoffen, wie Talk oder Siliciumdioxid, z.B. synthetischer amorpher Kieselsäure vom Typ Syloid® (Grace), z.B. SYLOID 244 FP, oder Netzmitteln, z.B. den weiter vorn genannten Polyäthylenglycolen oder Sorbaten, verwendet werden.

Elastische, filmähnliche Materialien sind insbesondere hydrophile, partiell verätherte Cellulosederivate.

Hydrophile, partiell verätherte Cellulosederivate sind z.B. Niederalkyläther der Cellulose mit einem durchschnittlichen molaren Substitutionsgrad (MS) grösser als eins und kleiner als drei und einem durchschnittlichen Polymerisationsgrad von ca. 100-5000.

Der Substitutionsgrad ist ein Mass für die Substitution der Hydroxygruppen durch Niederalkoxygruppen pro Glucoseeinheit. Der durchschnittliche molare Substitutionsgrad (MS) ist ein gemittelter Wert und gibt die Anzahl der Niederalkoxygruppen pro Glucoseeinheit im Polymerisat an.

Der durchschnittliche Polymerisationsgrad (DP) ist ebenfalls ein gemittelter Wert und gibt die durchschnittliche Anzahl an Glucoseeinheiten im Cellulosepolymerisat an.

Niederalkyläther der Cellulose sind z.B. Cellulosederivate, die an der Hydroxymethylgruppe (primäre Hydroxygruppe) der die Celluloseketten bildenden Glucoseeinheit und gegebenenfalls an der zweiten und dritten sekundären Hydroxygruppe durch C₁-C₄-Alkylgruppen, insbesondere Methyl oder Aethyl, oder durch substituierte C₁-C₄-Alkylgruppen, z.B. 2-Hydroxyäthyl, 3-Hydroxy-n-propyl, Carboxymethyl oder 2-Carboxyäthyl, substituiert sind.

Geeignete Niederalkyläther der Cellulose sind vorzugsweise Cellulosederivate, die an der Hydroxymethylgruppe (primäre Hydroxygruppe) der Glucoseeinheit durch die genannten C₁-C₄-Alkyl- oder durch substituierte C₁-C₄-Alkylgruppen und an der zweiten und gegebenenfalls dritten sekundären Hydroxygruppe durch Methyl- oder Aethylgruppen substituiert sind.

Geeignete Niederalkyläther der Cellulose sind insbesondere Methylcellulose, Aethylcellulose, Methylhydroxyäthylcellulose, Methylhydroxypropylcellulose, Aethylhydroxyäthylcellulose, Hydroxyäthylcellulose, Hydroxypropylcellulose, Carboxymethylcellulose (in Salzform z.B. als Natriumsalz) oder Methylcarboxymethylcellulose (ebenfalls in Salzform z.B. als Natriumsalz).

Bevorzugte Niederalkyläther der Cellulose sind Aethylcellulose (DP: ca. 150-1000, MS: ca. 1,2-1,8), z.B. vom Typ Aquacoat® (FMC Corp.), Hydroxyäthylcellulose (DP: ca. 120-1200, MS: ca. 1,2-2,5) und Hydroxypropylcellulose (DP: ca. 200-3000, MS: ca. 1,0-3,0).

Filmbildende Materialien mit Wasserdurchlässigkeit sind ferner Celluloseacetattrimellitat (CAT) oder Methacrylsäure-Methacrylat-1:1- oder -1:2-Copolymerisat, z.B. EUDRAGIT L und S, z.B. EUDRAGIT L 12.5 oder S 12.5.

Das filmbildende Material wird dabei als wässrige Dispersion von redispergierbarem Celluloseacetatphthalat - CAP - (Aquateric®:FMC), Polyvinylacetatphthalat - PVAP -(Coateric®: Colorcon), Hydroxypropylmethylcellulosephthalat - HPMCP (Aquacoat®HP 50 oder HP 55: Shin-Etsu) sowie insbesondere partiell durch C₁-C₄-Alkylgruppen verestertem Acrylsäure-Methacrylsäure-Copolymerisat aufgesprüht.

Ebenfalls wird ein partiell durch Methyl- und/oder Aethylgruppen verestertes Acrylsäure-Methacrylsäure-1:1-Copolymerisat vom Typ EUDRAGIT L 30 D oder wässrig dispergiertes EUDRAGIT L 100-55 verwendet.

Diese filmbildenden Materialien können zusätzliche Hilfsstoffe enthalten, z.B. wie Weichmacher z.B. Triäthylcitrat, z.B. Citroflex® (Pfizer), Triacetin, diverse Phthalate, z.B. Diäthyl- oder Dibutylphthalat, gemischte Mono- oder Diglyceride vom Typ Myvacet® (Eastman), z.B. MYVACET 9-40, die weiter vorn genannten Polyäthylenglycole, z.B. mit Molmasse von ca. 6000-8000 sowie Aethylenoxid-Propylenoxid-Blockcopolymere vom Typ Pluronic® (BASF) oder Synperonic® (ICI), pulverförmige Trennmittel, z.B. Magnesiumtrisilicat, Stärke oder synthetische, amorphe Kieselsäure vom Typ SYLOID, z.B. SYLOID 244 FP.

Die hydrophile, permeable innere Beschichtung b) enthält weisse Farbpigmente, vorzugsweise Titandioxid-Farbpigmente.

Das Ueberziehen der Tablettenkerne mit dem hydrophilen, permeablen Beschichtungsmittel erfolgt in an sich bekannter Weise unter Verwendung von üblichen Beschichtungsverfahren.

Beispielsweise wird das Beschichtungsmittel im gewünschten Mengenverhältnis in Wasser gelöst oder suspendiert. Gegebenenfalls setzt man Hilfsstoffe wie Polyäthylenglycol zu. Diese Lösung oder Dispersion wird auf die Tablettenkerne mit anderen Hilfsstoffen, z.B. Talk oder Siliciumdioxid, z.B. SYLOID 244 FP, aufgesprüht, z.B. unter Verwendung bekannter Verfahren, wie Sprühumhüllung in der Wirbelschicht z.B. in den Systemen von Aeromatic, Glatt, Wurster oder Hüttlin (Kugelcoater) sowie im Kessel nach den unter den Bezeichnungen Accela Cota oder Tauchrohrverfahren bekannten Verfahren.

Vorzugsweise wird eine wässrige Dispersion mit Hydroxypropylmethylcellulose (Cellulose HPMC) aufgesprüht.

Für die hydrophile, permeable äussere Beschichtung c), welche mit weissen Farbpigmenten, z.B. Titandioxid oder Talk, kombiniert mit Eisen-II-oxid-Farbpigmenten versehen ist, eignen sich ebenfalls die für die hydrophile, permeable innere Beschichtung b) genannten filmbildenden Beschichtungsmittel.

Vorzugsweise wird wie bei der inneren Beschichtung b) auch für die äussere Beschichtung c) eine wässrige Dispersion mit Hydroxypropylmethylcellulose (Cellulose HPM) aufgesprüht.

Die vorliegende Erfindung betrifft bevorzugt eine doppelt beschichtete Tablette für den Wirkstoff 10,11-Dihydro-10-oxo-5H-dibenz[b,f]azepin-5-carboxamid, welche durch folgende Bestandteile gekennzeichnet ist:
a) einen Tablettenkern enthaltend eine Dosiseinheit des Wirkstoffs 10,11-Dihydro-10-oxo-5H-dibenz[b,f]azepin-5-carboxamid;
b) eine hydrophile, permeable innere Beschichtung bestehend aus Hydroxypropylmethylcellulose und Polyethylenglycol 8000 enthaltend Titandioxidpigmente und
c) eine hydrophile, permeable äussere Beschichtung bestehend aus Hydroxypropylmethylcellulose und Polyethylenglycol 8000 enthaltend Titandioxidpigmente kombiniert mit Eisen-II-oxid-Farbpigmenten.

### Beispiele

### Beispiel 1

### Rezeptur:

| *Tablettenkern* | |
|---|---|
| TRILEPTAL Wirkstoff | 300,0 mg |
| AEROSIL® 200 (Silicaaerogel) | 2,5 mg |
| AVICEL® PH 102 (mikrokristalline Cellulose) | 85,0 mg |
| Cellulose HPM 603 (Hydroxypropylmethyl=cellulose) | 10,0 mg |
| Magnesiumstearat | 2,5 mg |
| NYMCEL® ZSB-10 (modifizierte Natriumcarboxy=methylcellulose niedrig substit.) | 40,0 mg |

| *Innenschicht* | |
|---|---|
| Cellulose HPM 603 (Hydroxypropylmethyl=cellulose) | ca. 6,9 mg |
| Polyethylenglycol 8000 | ca. 1,2 mg |
| Talkum PH | ca. 5,0 mg |
| Titandioxidpigment PH | ca. 1,9 mg |

| *Aussenschicht* | |
|---|---|
| Cellulose HPM 603 (Hydroxypropylmethyl=cellulose) | ca. 9,2 mg |
| Eisenoxid gelb 17268 | ca. 0,6 mg |
| Polyethylenglycol 8000 | ca. 1,7 mg |
| Talkum PH | ca. 6,6 mg |
| Titandioxidpigment PH | ca. 1,9 mg |
| Ungef.Gesamtgewicht | *475,0 mg* |

Der Wirkstoff TRILEPTAL und etwas Magnesiumstearat werden mit den übrigen Hilfsstoffen der weiter vorn für den Tablettenkern angegebenen Rezeptur im Container Mischer 20 min lang gemischt. Man kompaktiert das Gemisch in ca.2 - 6 mm starke Schülpen mit einer Prorosität von ca.17 - 21 %bei einem Pressdruck von ca.55 kN. Anschliessend mahlt man das kompaktierte Produkt zu Granulaten mit einer mittleren Teilchengrösse von ca.400 µm. Man gibt das restliche Magnesiumstearat zu dieser Mischung und mischt weitere 10 min. lang. Jeweils 440 mg der Rezepturmischung werden in die Matrize gefüllt und zu Tabletten mit einer Härte von ca. 125 N mit einer Exzentertablettiermaschine vom Typ Korsch DSKi 118 mit Pressdruckregistrierung und ovaloidem Stempel von 15 mm Länge und 6,5 mm Breite verarbeitet.

Die getrockneten Tablettenkerne werden anschliessend in der Wirbelschicht mit den in der Rezeptur genannten Hilfsstoffen beschichtet, wobei man als Lösungmittel eine wässrig äthnolische Mischung (11 % G/G Ethanol) verwendet, die ausserdem 5 % Isopropanol enthält.

Die beschichteten Tablettenkerne werden anschliessend mit der in der Rezeptur angegebenen Aussenschicht mit den genannten Farbpigmenten versehen, wobei man verfahrensmässig analog zur Herstellung der Innenschicht vorgeht.

## Patentansprüche

1. Doppelt beschichtete Tablette für den Wirkstoff 10,11-Dihydro-10-oxo-5H-dibenz[b,f]azepin-5-carboxamid gekennzeichnet durch
a) einen Tablettenkern enthaltend eine therapeutisch wirksame Dosis von 10,11-Dihydro-10-oxo-5H-dibenz[b,f]azepin-5-carboxamid;
b) eine hydrophile, permeable innere Beschichtung enthaltend weisse Farbpigmente und gegebenenfalls weitere Hilfsstoffe und
c) eine hydrophile, permeable äussere Beschichtung enthaltend weisse Farbpigmente kombiniert mit Eisen-II-oxid-Farbpigmenten und gegebenenfalls weitere Hilfsstoffe.

2. Doppelt beschichtete Tablette gemäss Anspruch 1, gekennzeichnet durch:
a) einen Tablettenkern enthaltend eine Dosiseinheit des Wirkstoffs 10,11-Dihydro-10-oxo-5H-dibenz[b,f]azepin-5-carboxamid;
b) eine hydrophile, permeable innere Beschichtung bestehend aus Hydroxypropylmethylcellulose und Polyethylenglycol 8000 enthaltend Titandioxidpigmente und
c) eine hydrophile, permeable äussere Beschichtung bestehend aus Hydroxypropylmethylcellulose und Polyethylenglycol 8000 enthaltend Titandioxidpigmente kombiniert mit Eisen-II-oxid-Farbpigmenten.

3. Verfahren zur Herstellung einer doppelt beschichteten Tablette für den Wirkstoff 10,11-Dihydro-10-oxo-5H-dibenz[b,f]azepin-5-carboxamid, dadurch gekennzeichnet, dass man
a) den Wirkstoff unter Zumischung von üblichen Hilfsstoffen und unter Anwendung üblicher Tablettierungsverfahren zu einem Tablettenkern verpresst und diesen nacheinander mit
b) einer hydrophilen, permeablen inneren Beschichtung mit weissen Farbpigmenten und
c) einer hydrophilen, permeablen äusseren Beschichtung mit weissen Farbpigmenten kombiniert mit Eisen-II-oxid-Farbpigmenten versieht.

## Claims

1. A double-coated tablet for the active ingredient 10,11-dihydro-10-oxo-5H-dibenzo[b,f]azepine-5-carboxamide, which comprises
a) a tablet core comprising a therapeutically effective dose of 10,11-dihydro-10-oxo-5H-dibenzo[b,f]azepine-5-carboxamide;
b) a hydrophilic, permeable inner coating comprising white pigments and, if desired, further excipients, and
c) a hydrophilic, permeable outer coating comprising white pigments combined with iron(II) oxide pigments and, if desired, further excipients.

2. A double-coated tablet according to claim 1, which comprises
a) a tablet core comprising a dosage unit of the active ingredient 10,11-dihydro-10-oxo-5H-dibenzo[b,f]azepine-5-carboxamide;
b) a hydrophilic, permeable inner coating consisting of hydroxypropylmethylcellulose and polyethylene glycol 8000 comprising titanium dioxide pigments, and
c) a hydrophilic, permeable outer coating consisting of hydroxypropylmethylcellulose and polyethylene glycol 8000 comprising titanium dioxide pigments combined with iron(II) oxide pigments.

3. A process for the preparation of a double-coated tablet for the active ingredient 10,11-dihydro-10-oxo-5H-dibenzo[b,f]azepine-5-carboxamide which process comprises
a) compressing the active ingredient, with the admixture of conventional excipients and using conventional tabletting methods, to form a tablet core and providing that core successively with
b) a hydrophilic, permeable inner coating comprising white pigments and
c) a hydrophilic, permeable outer coating comprising white pigments combined with iron(II) oxide pigments.

## Revendications

1. Un comprimé à double revêtement pour la substance active 10,11-dihydro-10-oxo-5H-dibenzo[b,f]azépine-5-carboxamide, caractérisé en ce qu'il comprend:
a) un comprimé-noyau comprenant une dose thérapeutiquement efficace du 10,11-dihydro-10-oxo-5H-dibenzo[b,f]azépine-5-carboxamide;
b) un enrobage interne hydrophile et perméable contenant des pigments blancs et éventuellement d'autres excipients, et
c) un enrobage externe hydrophile et perméable contenant des pigments blancs en association avec des pigments d'oxyde de fer (II) et éventuellement d'autres excipients.

2. Un comprimé à double revêtement selon la revendication 1, caractérisé en ce qu'il comprend
a) un comprimé-noyau contenant une dose unitaire de la substance active 10,11-dihydro-10-oxo-5H-dibenzo[b,f]azépine-5-carboxamide;
b) un enrobage interne hydrophile et perméable constitué d'hydroxypropylméthylcellulose et de polyéthylèneglycol 8000 contenant des pigments de dioxyde de titane, et
c) un enrobage externe hydrophile et perméable constitué d'hydroxypropylméthylcellulose et de polyéthylèneglycol 8000 contenant des pigments de dioxyde de titane en association avec des pigments d'oxyde de fer (II).

3. Un procédé de préparation d'un comprimé à double revêtement pour la substance active 10,11-dihydro-10-oxo-5H-dibenzo[b,f]azépine-5-carboxamide, caractérisé en ce que
a) on comprime la substance active ensemble avec des excipients habituels en un comprimé-noyau selon les méthodes de compression habituelles, et on fournit audit noyau successivement
b) un enrobage interne hydrophile et perméable contenant des pigments blancs, et
c) un enrobage externe hydrophile et perméable contenant des pigments blancs en association avec des pigments d'oxyde de fer (II).
